(19) Europäisches Patentamt
European Patent Office
Office européen des brevets

(11) Publication number: 0 070 088
A1

(12) # EUROPEAN PATENT APPLICATION

(21) Application number: 82302661.2

(22) Date of filing: 25.05.82

(51) Int. Cl.$^3$: C 07 D 233/64
C 07 D 263/12, C 07 D 277/10

(30) Priority: 26.05.81 GB 8115921
15.02.82 GB 8204409

(43) Date of publication of application:
19.01.83 Bulletin 83/3

(84) Designated Contracting States:
AT BE CH DE FR GB IT LI LU NL SE

(71) Applicant: BRIDGE CHEMICALS LIMITED
Old Powder Mills Nr. Leigh
Tonbridge Kent(GB)

(72) Inventor: Kelsey, Richard James
1 Fairlight Court Welland Road
Tonbridge Kent(GB)

(72) Inventor: Pearce, Andrew Kenneth
38 Burns Crescent
Tonbridge Kent(GB)

(72) Inventor: Rishman, Geoffrey
78 Mount Pleasant
Paddock Wood Kent(GB)

(72) Inventor: Sasse, Michael John
38 Bishop's Oak Ride
Tonbridge Kent(GB)

(74) Representative: Waters, David Martin, Dr. et al,
Smith Kline & French Laboratories Ltd. Patent
Department Mundells
Welwyn Garden City Hertfordshire AL7 1EY(GB)

(54) **Process for preparing an aminoethylthiomethylimidazole.**

(57) 4-(2-Aminoethylthiomethyl)-5-methylimidazole, an intermediate for the preparation of cimetidine, is prepared by the reaction of a 4,5-dimethylimidazole in which one methyl group is substituted by a hydroxy or lower alkoxy group or a chlorine or bromine atom, with a substituted ethyleneimino compound that is an N-(2-hydroxyethyl)-alkylthiocarboxamide, an N-(2-mercaptoethyl)-alkylcarboxamide, or a 2-alkyl-2-thiazoline.

EP 0 070 088 A1

Croydon Printing Company Ltd.

PROCESS FOR PREPARING AN AMINOETHYLTHIOMETHYLIMIDAZOLE

This invention relates to a process for preparing 4-(2-aminoethylthiomethyl)-5-methylimidazole, a chemical intermediate used in the commercial production of the drug cimetidine.

British Patent 1,338,169 discloses the preparation of 4-(2-aminoethylthiomethyl)-5-methylimidazole by the condensation of cysteamine with 4-hydroxymethyl-5-methylimidazole, and the conversion of the product to N-cyano-N'-methyl-N''-(2-[5(4)-methyl-4(5)-imidazolylmethylthio]ethyl)-guanidine (cimetidine) is described in British Patent 1,397,436.

A route used for the commercial production of cysteamine employs ethanolamine (1) as starting material and proceeds in four steps through N-hydroxyethylpropionamide (2), 2-ethyl-2-oxazoline (3) and N-mercaptoethylpropionamide (4) to cysteamine (5), as described in US Patent 4,086,274.

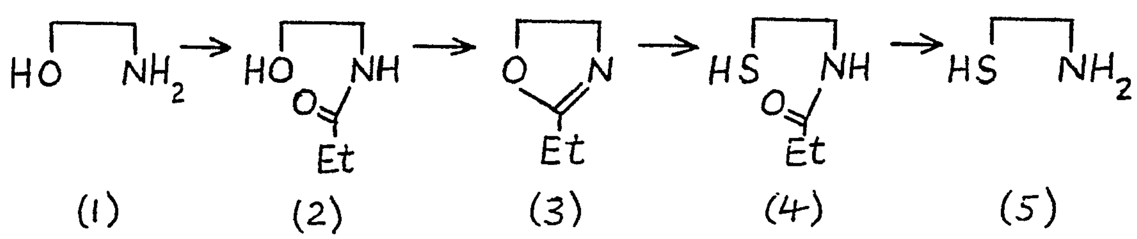

(1) (2) (3) (4) (5)

The cysteamine is produced as its hydrochloride from which water has to be driven off, leaving the molten hydrochloride, and because molten cysteamine hydrochloride sets to a glass on cooling and this is inconvenient for further processing, it is necessary to cool it on a rotating drum to obtain it in flake form.

It has now been found that the cysteamine stage can be avoided and processing made more economic by directly using intermediates preparable from a 2-alkyl-2-oxazoline that are cysteamine derivatives containing an organic substituent on the nitrogen atom, including N-mercapto-ethylpropionamide (4) itself.

The present invention provides a process for the preparation of 4-(2-aminoethylthiomethyl)-5-methyl-imidazole, in which an imidazole of the structure (6)

(6) 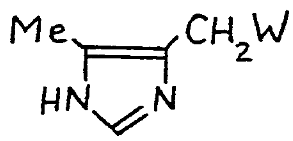

where W is a hydroxy group or an alkoxy group having from 1 to 6 carbon atoms, or a chlorine or bromine atom, is reacted with an ethyleneimino compound of the structure $XCH_2CH_2NY\underset{R}{C}Z$, where X is a hydroxy group, Y is H and Z is a sulphur atom; or X is a mercapto group, Y is H and Z is an oxygen atom; or Y is a double bond and X and Z together represent a sulphur atom; and R is an alkyl group having from 1 to 4 carbon atoms.

Where X is a hydroxy group, Y is H, and Z is a sulphur atom, the ethyleneimino compound is an N-(2-hydroxyethyl)-alkylthiocarboxamide. Where X is a mercapto group, Y is H, and Z is an oxygen atom, the ethyleneimino compound is an N-(2-mercaptoethyl)-alkylcarboxamide. Where Y is a double bond and X and Z together represent a sulphur atom, the ethyleneimino compound is a 2-alkyl-2-thiazoline. These compounds have in common an organic substituent on the imino group that is not present in cysteamine. The structures of the three types of ethyleneimino compound used in the process are shown in the following diagram as (7), (8) and (9).

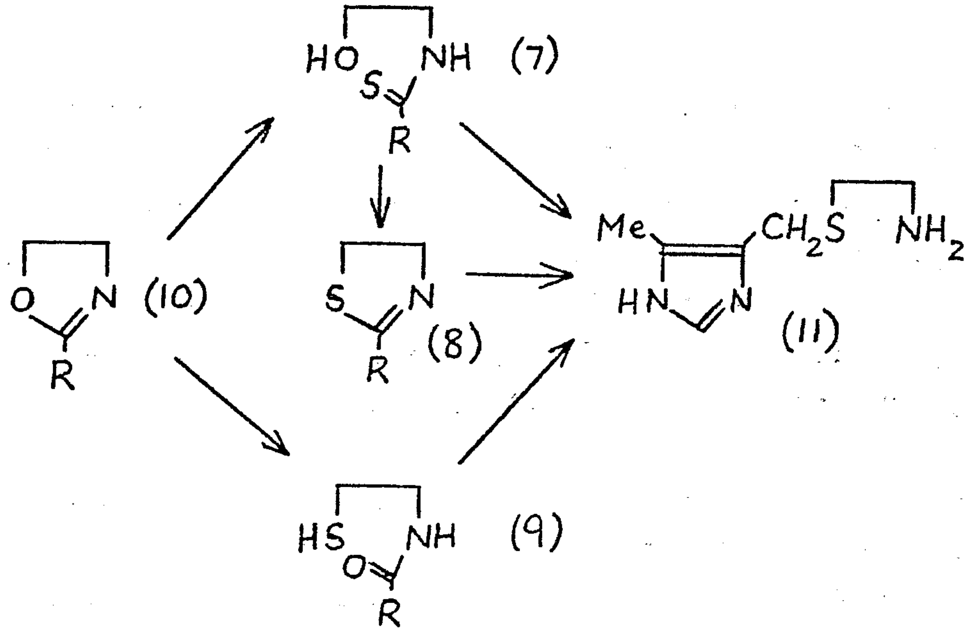

The N-(2-hydroxyethyl)-alkylthiocarboxamide (7) can be prepared in high yield from the 2-alkyl-2-oxazoline (10) and can either be used directly in the process of the invention or can be cyclised to the 2-alkyl-2-thiazoline (8) which is then used in the process. The N-(2-mercaptoethyl)-alkylcarboxamide (9) can be prepared from the 2-alkyl-2-oxazoline (10) as described in USP 4,086,274.

The conversion of the compounds (7), (8) and (9) to 4-(2-aminoethylthiomethyl)-5-methylimidazole (11) can be effected in high yield. Both routes (10) through (7) to (11), and (10) through (9) to (11) have the advantage that besides avoiding formation of cysteamine hydrochloride, they employ only two chemical process steps starting with the oxazoline instead of three when the cysteamine route is used. The process using the route (10) through (7) to (11) is particularly advantageous, as it has been found that the intermediate (7) can be prepared readily using undried oxazoline (10) containing a molar equivalent of water produced in the cyclisation step by which (10) is prepared from an N-(2-hydroxyethyl)-alkylcarboxamide such as the compound (2), whereas this is not possible using the route (10) through (9) to (11), because water interferes with the conversion of (10) to (9).

In a process of the invention preferably W in the imidazole of structure (6) is a hydroxy group; where it is an alkoxy group, preferably it is methoxy, ethoxy or n-butoxy. The imidazole can be used as the free base, or as a salt, for instance the hydrochloride. The group R in the ethyleneimino compound is preferably a straight-chain alkyl group, especially methyl or ethyl.

The process of the invention is carried out under hydrolytic conditions that remove the organic group attached to the nitrogen atom and containing R, so that the cysteamine is generated in situ. This can be effected by the action of a strong mineral acid, for instance concentrated aqueous hydrochloric or hydrobromic acid. Thus a mixture of the organic reagents and the hydrolysing acid on heating to from $50^{\circ}$ to $150^{\circ}C$, under reflux if required, results in condensation with removal of the organic group containing R and formation of the imidazolylmethyl thioether product (11). As the imidazole (6) is more expensive than the ethyleneimino compound, it is preferable to use from 1 to 1.3 molar equivalents of the ethyleneimino compound to 1 molar equivalent of the imidazole. Where the mercaptoethyl compound (9) is used the best yields are obtainable with from 1 to 1.1 molar equivalents of it to 1 molar equivalent of the imidazole. The product can be isolated as the free base, but preferably it is isolated as a crystalline salt with an acid, for example as the dihydrochloride or dihydrobromide. A total of from 2 to 2.5 molar equivalents of hydrogen halide to 1 of imidazole can be used to produce the dihydrohalide.

Preferably where the ethyleneimino compound is an N-(2-hydroxyethyl)-alkylthiocarboxamide, the process is one in which it has been prepared by the reaction of

hydrogen sulphide with a 2-alkyl-2-oxazoline in the presence of an unreactive organic base, especially a tertiary nitrogen base, for instance triethylamine, tri-n-propylamine or 1,5-diazabicyclo(4,5,0)undec-5-ene, preferably at a temperature in the range from 50° to 100°C. Especially convenient as starting material is the 2-alkyl-2-oxazoline obtained from cyclisation of an N-(2-hydroxymethyl)-alkylcarboxamide without removal of the water eliminated on cyclisation, for instance as described in USP 3,681,329 and USP 4,203,900. It has been discovered that in the presence of the base the addition of the hydrogen sulphide is directed to produce the thiocarboxamide. Preferably from 0.1 to 0.5 molar equivalents of base to 1 of thiocarboxamide are used.

Where the ethyleneimino compound is an N-(2-mercaptoethyl)-alkylcarboxamide, the starting material can be obtained as described in USP 4,086,274.

Where the ethyleneimino compound is a 2-alkyl-2-thiazoline, it can be obtained from the corresponding N-(2-hydroxyethyl)-alkylthiocarboxamide by cyclodehydration, for instance by heating with a dehydration catalyst, especially a sulphonic acid, for example methanesulphonic, ethanesulphonic or toluene-p-sulphonic acid. It is convenient to dissolve the thiocarboxamide in an inert solvent, for instance dichloromethane, with the dehydration catalyst and to heat the mixture at from 30° to 100°C until reaction is complete.

The invention is illustrated by the following Examples, in which temperatures are in °C, and parts are by weight.

## EXAMPLE 1

a. Hydrogen sulphide gas was passed for 4 hr into a mixture of 2-methyl-2-oxazoline (42.5 g, 0.5 mol) and triethylamine (5.05 g, 0.05 mol). At the start the mixture was heated to bring it to 60° in 10 min and to maintain it at that temperature for 20 min: an exothermic reaction maintained it at 60° until near the end of the period, when heating was reapplied to maintain the temperature. Excess hydrogen sulphide was purged with nitrogen, and triethylamine was removed under reduced pressure at 25° to give N-(2-hydroxyethyl)-thioacetamide as a yellow liquid of purity (hplc) 98.3% (58.5 g, 96.6%).

b. To N-(2-hydroxyethyl)-thioacetamide (11.2 g, 0.094 mol) was added concentrated hydrochloric acid (15 ml), followed by 4-hydroxymethyl-5-methylimidazole hydrochloride (12.6 g, 0.075 mol), and the resulting brown solution was heated under reflux for 18 hr. Volatile materials were removed by distillation until the residue reached 160°, and the residue was allowed to cool to 90°, hot glacial acetic acid (40 ml) was added and the mixture seeded with crystals of end-product and allowed to cool. After standing overnight the crystalline product was filtered off, washed with isopropyl alcohol (25 ml) and solvent removed at 105° to give 4-(2-aminoethylthiomethyl)-5-methylimidazole dihydrochloride (13.9 g, 76.0% based on hydroxymethylimidazole), m.p. 188-90°.

## EXAMPLE 2

a(i). Hydrogen sulphide gas was passed for 5 hr into a mixture of 2-ethyl-2-oxazoline (49.5 g, 0.5 mol) and 1,5-diazabicyclo(4,5,0)undec-5-ene (15.2 g, 0.1 mol). The mixture was heated to and maintained at 60° as in

Example 1a. The yellow reaction mixture was purged with nitrogen to remove excess hydrogen sulphide, poured on to a column of silica and chromatographed with ethyl acetate as eluent. Fractional distillation of the eluate gave N-(2-hydroxyethyl)-thiopropionamide as a straw coloured liquid, b.p. 136°/0.15 mm Hg.

a(ii). Hydrogen sulphide was passed for 3 hr into a mixture of 2-ethyl-2-oxazoline (49.5 g, 0.5 mol) and triethylamine (20.2 g, 0.2 mol). The temperature of the mixture was brought to and maintained at 60° as before, excess hydrogen sulphide was then removed by purging with nitrogen, and the triethylamine was removed by evaporation under reduced pressure at 25° to give N-(2-hydroxyethyl)-thiopropionamide (66.0 g, 99.4%) as a yellow liquid.

a(iii). A process was carried out as in a(ii), except that the 2-ethyl-2-oxazoline starting material used contained in addition 9 g water and was typical of the wet ethyloxazoline obtained directly by cyclodehydration of N-(2-hydroxyethyl)-propionamide. The yield of product was 64.2 g (96.6%).

b. To N-(2-hydroxyethyl)-thiopropionamide (33.2 g, 0.25 mol) was added concentrated hydrochloric acid (39 ml), followed by 4-hydroxymethyl-5-methylimidazole hydrochloride (29.7 g, 0.20 mol) and the resulting brown mixture heated under reflux for 18 hr. The product was treated as in Example 1b and there was obtained 4-(2-aminoethylthiomethyl)-5-methylimidazole dihydrochloride (43.0 g, 88.1% based on hydroxymethylimidazole), m.p. 188-90°.

EXAMPLE 3

4-Hydroxymethyl-5-methylimidazole hydrochloride (17.58 parts), N-(2-mercaptoethyl)-propionamide (15.75 parts) and concentrated hydrochloric acid (13.0 parts) were heated together under reflux for 15 hr. Propionic and hydrochloric acids were then removed by distillation until the residue reached 150$^{o}$, and glacial acetic acid (72 parts) was added, the temperature of the mixture being maintained at above 100$^{o}$ throughout the addition. After cooling the mixture to 50$^{o}$ a seeding amount of crystalline end-product was added, the mixture was stirred and maintained at 45-50$^{o}$ for 30 min and then cooled to below 20$^{o}$ and stirred for 1 hr to complete crystallisation. The product was separated by centrifugation, washed 3 times in the centrifuge with isopropyl alcohol, and dried at 100$^{o}$ to give 4-(2-aminoethylthiomethyl)-5-methylimidazole dihydrochloride (27 parts, 93%), m.p. 188-90$^{o}$.

EXAMPLE 4

a. Methanesulphonic acid (1.0 g, 0.01 mol) was added to a solution of N-(2-hydroxyethyl)-thiopropionamide (1.0 g, 0.0075 mol) in dichloromethane (5 ml) and the mixture heated under reflux (42$^{o}$) for 24 hr. After cooling to 0$^{o}$ water (5 ml) was added, and then solid potassium carbonate until the aqueous phase had pH 9. The aqueous and non-aqueous phases were separated, the aqueous phase extracted with dichloromethane (5 ml), and the extracts added to the non-aqueous phase, which was then dried with calcium chloride and solvent removed by evaporation at ambient temperature to give 2-ethyl-2-thiazoline (0.54 g, 62%) b.p. 165-7$^{o}$.

b. Concentrated hydrochloric acid (36 ml) was added slowly to 2-ethyl-2-thiazoline (37.95 g, 0.33 mol), followed by 4-hydroxymethyl-5-methylimidazole hydrochloride (49 g, 0.33 mol), and the mixture heated under reflux for 16 hr. The product was subjected to distillation until the residue reached 145°, hot glacial acetic acid (220 ml) was added to the residue and the mixture was cooled to 60° and seed crystal of the end-product was added. The resulting suspension was cooled to 20° and stirred for 1 hr, the crystalline precipitate filtered off, washed with isopropyl alcohol (100 ml) and solvent removed by evaporation at 105° to give 4-(2-aminoethylthiomethyl)-5-methyl-imidazole dihydrochloride (64.4 g, 79.3%), m.p. 188-90°.

## EXAMPLES 5 to 8

4-(2-Aminoethylthiomethyl)-5-methylimidazole dihydrochloride is prepared as described in Examples 1b, 2b, 3 and 4b, except that instead of 4-hydroxymethyl-5-methylimidazole hydrochloride there is used 4-methoxymethyl-5-methylimidazole hydrochloride (13.8 g, 32.5 g, 19.2 parts and 41.5 g), respectively.

## EXAMPLES 9 to 12

4-(2-Aminoethylthiomethyl)-5-methylimidazole dihydrochloride is prepared as described in Examples 1b, 2b, 3 and 4b, except that instead of 4-hydroxymethyl-5-methylimidazole hydrochloride there is used 4-chloromethyl-5-methylimidazole hydrochloride (34.6 g, 33.4 g, 19.7 parts and 42.7 g), respectively.

EXAMPLES 13 to 16

Processes are carried as described in Examples 1b, 2b, 3 and 4b, except that instead of hydrochloric acid and 4-hydroxymethyl-5-methylimidazole hydrochloride there are used hydrobromic acid and 4-bromomethyl-5-methylimidazole hydrobromide (21.7 g, 51.2 g, 30.3 parts and 65.4 g), respectively, to obtain 4-(2-aminoethylthiomethyl)-5-methylimidazole dihydrobromide, m.p. 208-211°.

## CLAIMS

1. A process for the preparation of 4-(2-aminoethylthiomethyl)-5-methylimidazole, in which an imidazole of the structure

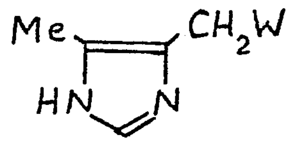

where W is a hydroxy group or an alkoxy group having from 1 to 6 carbon atoms or a chlorine or bromine atom, is reacted with a compound of the structure $XCH_2CH_2NY\underset{R}{C}Z$, where X is a hydroxy group, Y is H, and Z is a sulphur atom; or X is a mercapto group, Y is H and Z is an oxygen atom; or Y is a double bond and X and Z together represent a sulphur atom; and R is an alkyl group having from 1 to 4 carbon atoms.

2. A process according to claim 1, where R is a methyl or ethyl group.

3. A process according to Claim 2, where W is a hydroxy group.

4. A process according to any preceding claim, where the reaction is effected with concentrated hydrochloric acid.

5. A process according to any preceding claim, where the ethyleneimino compound is an N-(2-hydroxyethyl)-alkylthiocarboxamide.

6. A process according to Claim 5, where the thiocarboxamide has been prepared by the reaction of hydrogen sulphide with a 2-alkyl-2-oxazoline in the presence of an unreactive organic base.

7. A process according to Claim 6, where the 2-alkyl-2-oxazoline employed has been obtained by cyclisation of an N-(2-hydroxyethyl)-alkylcarboxamide without removal of the water eliminated on cyclisation.

8. A process according to any one of Claims 1 to 4, where the ethyleneimino compound is an N-(2-mercaptoethyl)-alkylcarboxamide.

9. A process according to any one of Claims 1 to 4, where the ethyleneimino compound is a 2-alkyl-2-thiazoline.

10. A process according to Claim 9, where the thiazoline has been prepared by the cyclodehydration of an N-(2-hydroxyethyl)-alkylthiocarboxamide.

0070088

European Patent Office

# EUROPEAN SEARCH REPORT

Application number

EP 82 30 2661

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 006 102 (BASF)<br>--- | | C 07 D 233/64<br>C 07 D 263/12<br>C 07 D 277/10 |
| A | US-A-3 950 353 (DURANT et al.)<br>--- | | |
| A | FR-A-2 418 227 (DEVINTER)<br>--- | | |
| P,A | GB-A-2 079 276 (SOGO)<br>----- | | |
| | | | TECHNICAL FIELDS SEARCHED (Int. Cl. 3) |
| | | | C 07 D 233/00 |

The present search report has been drawn up for all claims

| Place of search | Date of completion of the search | Examiner |
|---|---|---|
| THE HAGUE | 06-09-1982 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document